# EUROPEAN PATENT APPLICATION

(11) **EP 1 378 258 A1**
(43) Date of publication of application: **07.01.2004**
(21) Application number: 02713297.6
(22) Date of filing: 05.04.2002
(51) Int. Cl.: A61M 1/36, B01D 15/04, B01J 20/26

(54) **ADSORBENT DEVICE FOR BODY FLUID TREATMENT**

(30) Priority: 12.04.2001 JP 2001113643
(71) Applicant: Kuraray Co., Ltd., Kurashiki-City, Okayama 710-8622 (JP)
(72) Inventor: HAYASHI, Nobuyuki, Chuo-ku, Tokyo 103-8254 (JP); NAKAJI, Shuhei, Kurashiki-shi, Okayama 710-8622 (JP)
(74) Representative: VOSSIUS & PARTNER
(86) International application number: PCT/JP2002/003425
(87) International publication number: WO 2002/083202

(57) **Abstract**

The above-mentioned problems can be solved by an adsorber for body fluid treatment packed with an adsorbent coated with a hydrophilic polymer, which includes an adsorbent packing part not filled with a filling liquid, particularly the above-mentioned adsorber for body fluid treatment, wherein the adsorbent contains two or more kinds of metal salt type cation-exchange resins that are different from each other.

## Description

### Technical Field

The present invention relates to an adsorber for body fluid treatment. The adsorber for body fluid treatment of the present invention is superior in safety and operability, and is particularly useful for appropriately maintaining potassium ion concentration of preserved blood for transfusion, since it easily and certainly removes potassium ion from preserved blood for transfusion, which contains high concentration potassium ion.

### Background Art

Conventionally, adsorbers for body fluid treatment, such as an adsorber for blood purification therapy and an adsorber for plasma perfusion and the like, are considered to require a complicated degassing operation to remove air bubbles present between adsorbents or within adsorbents in a filling liquid in the adsorber, thereby to prevent undesirable entry of air into the body. To simplify such complicated operation, an adsorber having an adsorbent packing part filled with a filling liquid has been used (e.g., JP-A-2-77266).

In an adsorber having an adsorbent packing part filled with a filling liquid, however, the adsorbent stays in contact with the filling liquid for a long time, which facilitates elution of the substances in the adsorbent into the filling liquid, thereby possibly contaminating body fluids such as blood, plasma and the like during use. Even if the adsorber is washed, there still remains a possibility of undesirable entry of the filling liquid in the adsorber into the body fluid, which contains the eluted substances, because the eluted substances may not be completely washed away.

To prevent elution of substances, therefore, the use of an adsorber having an adsorbent packing part not filled with a filling liquid, or an adsorber packed with a dry adsorbent without using a filling liquid is considered to be desirable. When a general adsorbent whose surface is not coated with a hydrophilic polymer is used, the adsorbability that the adsorbent inherently possesses cannot be exhibited unless an adsorbent packing part is filled with a filling liquid. This is because air bubbles are present between the adsorbents, and particularly in the case of porous adsorbent, many air bubbles are also present in the pores therein, which prevents sufficient penetration of body fluid between adsorbents or within pores, making insufficient contact between adsorbent and body fluid. An adsorber packed with the above-mentioned dry adsorbent without using a filling liquid cannot exhibit the adsorbability it inherently possesses for the same reasons as given above. In addition, an excessive pressure needs to be applied on an adsorber inlet to flow the body fluid into the adsorber.

While the above-mentioned adsorber for body fluid treatment has been mainly used for blood purification therapy, it has not yet been generally used in the transfusion field.

In recent years, the risk of induction of Graft-versus-Host Disease (GVHD) after transfusion, due to active leukocytes in blood preparations, has been clarified. In addition, the risk of infection with virus contaminating blood preparations has been pointed out for some time. As a countermeasure, irradiation on blood preparations is becoming increasingly common to eradicate lymphocytes in leukocyte and virus contained in blood preparations.

However, irradiated blood preparations sometimes show increased potassium ion concentration due to the potassium ion effluxed from cell components destroyed by radiation. The destruction of cell also proceeds during storage of blood preparations, whereby the potassium ion concentration still increases. When such blood preparations are transfused, hyperkalemia is caused, which may even result in a cardiac arrest. To avoid this, irradiation is preferably conducted immediately before use, but it is often clinically difficult to do so. Thus, provision of a transfusion device that ensures safety of transfusion has been desired, which removes potassium ion immediately before transfusion to prevent hyperkalemia after transfusion.

Heretofore, as a material for controlling ion concentration in blood, there are known a transfusion set including a strong acidic ion-exchange resin to adsorb potassium ion in blood in the circuit of transfusion (JP-A-52-79594), an apparatus for controlling ion concentration of blood preparations, including a cation-exchange resin (JP-A-7-284532), and a preserved blood transfusion system using an ion-exchange resin (JP-A-7-308368). However, it has been pointed out that these ion concentration controlling materials control concentration of the objective ion, but at the same time, vary concentrations of other ions that play important roles in the body.

To solve this problem, a potassium ion remover to selectively remove potassium ion in blood has been proposed, which comprises a sodium salt type ion-exchange resin and a calcium salt type ion-exchange resin (JP-A-5-31185). This potassium ion remover is preferable when potassium ion is gradually removed from the circulating blood to lower the potassium ion concentration with the lapse of time, as in the blood purification therapy. However, it is hardly adequate to say that the remover is capable of sufficiently removing only potassium ion, when high concentration potassium ion needs to be removed constantly as in the case of preserved blood for transfusion.

In view of the problems of these conventional techniques, the present invention aims at providing an adsorber for body fluid treatment, which permits only a small amount of eluate, easy wetting and easy handling, and an adsorber for body fluid treatment, which can certainly remove, by adsorption, potassium ion in the preserved blood for transfusion.

### Disclosure of the Invention

According to the present invention, the above-mentioned object can be achieved by providing an adsorber for body fluid treatment packed with an adsorbent coated with a hydrophilic polymer, which comprises an adsorbent packing part not filled with a filling liquid, particularly such an adsorber for body fluid treatment, wherein the above-mentioned adsorbent comprises two or more kinds of metal salt type cation-exchange resins that are different from each other. In the present invention, by the "body fluid" is meant a liquid component derived from an organism such as blood, plasma, ascites and the like. The adsorber for body fluid treatment of the present invention can treat any of these, but it is preferably used for the treatment of blood from among these.

Accordingly, the present invention provides the following.
[1] An adsorber for body fluid treatment packed with an adsorbent coated with a hydrophilic polymer, which comprises an adsorbent packing part not filled with a filling liquid.
[2] The adsorber for body fluid treatment of the above-mentioned [1], wherein the adsorbent is wet with a filling liquid but the adsorbent packing part is not filled with a filling liquid.
[3] The adsorber for body fluid treatment of the above-mentioned [2], wherein a ratio of a distance between the bottom of the packing part and a liquid surface of the filling liquid to a distance between the bottom of the packing part and the top of the adsorbent is not more than 0.8.
[4] The adsorber for body fluid treatment of any of the above-mentioned [1] to [3], wherein the adsorbent comprises two or more kinds of metal salt type cation-exchange resins that are different from each other.
[5] The adsorber for body fluid treatment of the above-mentioned [4], wherein the adsorbent comprises 90-99.9 parts by volume of a sodium salt type cation-exchange resin and 10-0.1 part by volume of a calcium salt type cation-exchange resin.
[6] The adsorber for body fluid treatment of any of the above-mentioned [1] to [5], which is an ion concentration controller of preserved blood for transfusion.

### Detailed Description of the Invention

In the following, the present invention is explained in detail.

The adsorbent in the present invention is coated with a hydrophilic polymer. The hydrophilic polymer is a water soluble polymer, a water swellable polymer or a water wettable polymer, which comprises, as a functional group, a dissociative group such as carboxyl group, sulfonic acid group, quaternary amine group and the like, or a hydrophilic non-dissociative group such as hydroxyl group, acrylamide group, ether group and the like. Specific examples thereof include acrylate, methacrylate, polyvinyl alcohol, ethylene-vinyl alcohol, cellulose, ethylene glycol and vinyl pyrrolidone polymers, and the like. Of these, methacrylate polymer is preferable and polyhydroxyethyl methacrylate is particularly preferable.

Such hydrophilic polymer shows high affinity for water or body fluid. An adsorbent coated with the polymer easily becomes wet with liquid upon contact therewith, such as water, body fluid and the like, and absorbs water to become wet, whereby an adsorbent not in a wet state can be rapidly made wet. In addition, an adsorbent coated with a hydrophilic polymer resists coagulation, hemolysis, adhesion of blood cells or plasma protein, and the like and is superior in biocompatibility.

The adsorbent coated with a hydrophilic polymer can be produced by immersing an adsorbent material in a hydrophilic polymer solution, followed by drying and solidifying. The material of an adsorbent may be any of organic substances and inorganic substances, and is preferably an organic substance capable of taking any shape and having a high strength. Specific examples of the organic substances include cellulose, polyvinyl alcohol, polyacrylamide, agarose, phenol, amide, styrene-divinylbenzene substances and the like.

The above-mentioned organic substance to be used as a material of an adsorbent is more preferably a cation-exchange resin. The ion-exchange resin here has a structure wherein an exchange group, which is ion-exchangeable, is stably bonded via a covalent bond to a polymer matrix having three-dimensional bonds. The polymer matrix having three-dimensional bonds is not particularly limited, and a broad range of polymers capable of stably introducing ion-exchange groups can be used as long as they have three-dimensional bonds. Examples thereof include styrene-divinylbenzene copolymer, (metha)acrylic polymer, phenolic polymer, amide polymer and the like. Of these, styrene-divinylbenzene copolymer is more preferable, because it shows high chemical stability of the polymer itself, high chemical stability and ion-exchange capacity of the polymer, into which an ion-exchange group has been introduced, and it can be easily made into uniform spherical particles by pearl polymerization.

The above-mentioned cation-exchange resin is obtained by introducing an acidic ion-exchange group into the above-mentioned polymer matrix. As the ion-exchange group, a broad range of acidic groups can be used, such as sulfonic acid group, carboxyl group and the like. Of these, sulfonic acid group is preferable.

The ion-exchangeable hydrogen atom in the cation-exchange resin can be converted to sodium atom, calcium atom, magnesium atom and the like, and the ion-exchange resin can be used as various metal salt types such as sodium salt type, calcium salt type, magnesium salt type and the like. In these metal salt type ion-exchange resins, sodium atom, calcium atom or magnesium atom is bound to an acidic ion-exchange group via an ionic bond, and is easily exchanged with a different cation present in an aqueous solution or body fluid.

The material of the adsorbent used in the present invention preferably comprises two or more kinds of metal salt type cation-exchange resins that are different from each other, from the aspect of a potassium adsorption effect. It is more preferable that it comprises a sodium salt type (Na salt type) cation-exchange resin and a calcium salt type (Ca salt type) cation-exchange resin. When the ratio of the Ca salt type ion-exchange resin in the above-mentioned cation-exchange resin is too small, a concentration of calcium ion in the blood treated by an adsorber for body fluid treatment becomes low, which may cause a blood coagulation type functional disorder such as difficult coagulation of blood and the like. When the ratio of the Ca salt type ion-exchange resin in the above-mentioned cation-exchange resin is too high, a calcium ion concentration of blood becomes high, posing a risk of incident of alkalosis where the blood shows an alkaline pH. Thus, the ratio of the Na salt type cation-exchange resin and Ca salt type cation-exchange resin is preferably in the range of Na salt type/Ca salt type = 90-99.9 parts by volume/10-0.1 part by volume, more preferably in the range of Na salt type/Ca salt type = 98-99.9 parts by volume/2-0.1 part by volume. As used herein, the volume of an ion-exchange resin as expressed by "part(s) by volume" means an apparent volume of the ion-exchanqe resin in a wet state, which is specifically measured by settling the ion-exchange resin in a graduated cylinder filled with water and determining the volume. To prepare an Na salt type cation-exchange resin and a Ca salt type cation-exchange resin at a predetermined mixing ratio, for example, the volume of each cation-exchange resin is measured with a graduated cylinder by the above-mentioned method and these resins are mixed in water.

As the above-mentioned polymer matrix of Na salt type cation-exchange resin, the polymer matrix of Ca salt type cation-exchange resin, and the acidic ion-exchange group, one kind or two or more kinds may be used, but as the cation-exchange resin, one having almost the same ion-exchange capacity per unit volume is preferably used in view of handling.

The ion-exchange resin to be used in the present invention is preferably a strong acidic porous cation-exchange resin comprising a styrene-divinylbenzene copolymer as a polymer matrix and a sulfonic acid group as an acidic exchange group, which is preferably exemplified by one obtained by converting an ion-exchange resin commercially available as DIAION RCP-160M (manufactured by Mitsubishi Chemical Corporation) to the Na salt type resin and the Ca salt type resin and mixing them.

The adsorbent to be coated with a hydrophilic polymer may have any shape such as sheet, fiber, spherical particle, pulverized particle, hollow fiber and the like. Of these, an adsorbent having a shape of spherical particle is preferable, since it enlarges the contact area with the substances to be adsorbed and enhances adsorbability. In addition, spherical particles include those of a non-porous gel type and those of a porous type. In the present invention, both types can be used. To enhance the above-mentioned adsorbability, those of a porous type are preferable.

The adsorber for body fluid treatment of the present invention can be produced by a method comprising packing a container, such as a column and the like, with the adsorbent coated with the above-mentioned hydrophilic polymer, a method comprising packing a container, such as a column and the like, containing a filling liquid, with the adsorbent to force out the filling liquid, and the like. Conventional adsorbers comprise an adsorbent packing part filled with a filling liquid, but the adsorber for body fluid treatment of the present invention comprises a packing part for the adsorbent coated with the above-mentioned hydrophilic polymer, which is not filled with a filling liquid. Here, by the phrase of "an adsorbent packing part not filled with a filling liquid" is meant that the liquid face of the filling liquid in an adsorbent packing part is lower than the top of the adsorbent, in other words, that the adsorbent is not completely immersed in the filling liquid. The proportion of the adsorbent not immersed in the filling liquid, as expressed by a ratio of a distance between the bottom of the packing part and a liquid surface of the filling liquid to a distance between the bottom of the packing part and the top of the adsorbent, wherein the adsorbent is packed in the packing part such that the top is horizontal, is preferably not more than 0.8, more preferably not more than 0.5. As the filling liquid, liquids such as water, physiologic saline, phosphate buffer, citrate buffer and the like are used. From among these, water is most frequently used, in view of easy availability, safety and cost. As the kind of water, there are mentioned distilled water, ion-exchanged distilled water, reverse osmosis membrane filtrate water and the like, with preference given to ion-exchanged distilled water. In the present invention, the adsorbent is wetted with the above-mentioned liquid to prevent elution of substances from the adsorbent to the filling liquid. The adsorbent packing part is preferably not filled with a filling liquid, more preferably a filling liquid is not contained in the adsorbent packing part.

By bringing the adsorber for body fluid treatment of the present invention into contact with a body fluid, the body fluid can be purif ied. The adsorber for body fluid treatment of the present invention is particularly suitable for removing potassium ion from preserved blood for transfusion containing high concentration potassium ion, and appropriately maintaining the potassium ion concentration of the preserved blood for transfusion. As used herein, by the preserved blood for transfusion is meant blood preparations, such as a whole blood preparation, an erythrocyte preparation, a concentrated erythrocyte preparation and the like, which is generally supplied as a 200 mL or 400 mL blood bag, with 200 mL as one unit.

The adsorber for body fluid treatment of the present invention is preferably used upon sterilization, wherein the sterilization method may be autoclave sterilization, radiation sterilization and the like.

### Examples

The present invention is explained by referring to the following Examples, which are not to be construed as limitative.

### Production Example 1

(1) A sulfonic acid type strong acidic cation-exchange resin (hydrogen type) [DIAION RCP-160M (manufactured by Mitsubishi Chemical Corporation)] comprising a styrene-divinylbenzene copolymer as a polymer matrix was treated with an aqueous sodium chloride solution according to a conventional method to give an Na salt type cation-exchange resin, wherein a counter ion was of an Na salt type. In the same manner, RCP-160M was treated with an aqueous calcium chrolide solution according to a conventional method to give a Ca salt type cation-exchange resin, wherein a counter ion was of a Ca salt type. The obtained Na salt type cation-exchange resin and Ca salt type cation-exchange resin were mixed at a ratio of Na salt type/Ca salt type = 94 parts by volume/6 parts by volume to give a mixture of an Na salt type cation-exchange resin and a Ca salt type cation-exchange resin.
(2) A mixture of hydroxyethyl methacrylate (99.5 parts by weight), glycidyl methacrylate (0.5 part by weight), diisopropylperoxy dicarbonate (0.1 part by weight) and 95% ethanol (700 parts by weight) was stirred in nitrogen at 60°C for 8 hr to give polyhydroxyethyl methacrylate having glycidyl groups. This polymer was dissolved in 95% ethanol to give a solution having a concentration of 0.75 wt%. The mixture of cation-exchange resin prepared in the above-mentioned (1) was immersed in this solution at room temperature for 4 hr and taken out. After air drying overnight, the mixture was dried with hot air at 80°C for 5 hr and heat treated at 110°C for 2 hr. This operation was repeated twice to give an adsorbent coated with polyhydroxyethyl methacrylate (hereinafter to be sometimes referred to as PHEMA).

### Example 1

The adsorbent obtained in Production Example 1 in a dry state was measured (16 mL) with a graduated cylinder, and then the adsorbent was packed in a polypropylene column to give an adsorber containing a dry adsorbent, which was subjected to radiation sterilization.

### Example 2

The adsorbent obtained in Production Example 1 was immersed in ion-exchanged distilled water and degassed under reduced pressure. The adsorbent obtained by this step, which was wet with ion-exchanged distilled water, was measured for a necessary amount (20 mL) with a graduated cylinder filled with ion-exchanged distilled water, and then the adsorbent was packed in a polypropylene column filled with ion-exchanged distilled water. Compressed air (1 kg/cm²) was fed into the column for 1 min to force out the filling liquid to give an adsorber containing an adsorbent in a wet state and having a packing part free of a filling liquid, which was subjected to radiation sterilization.

### Example 3

The adsorbent obtained in Production Example 1 was immersed in ion-exchanged distilled water and degassed under reduced pressure. The adsorbent obtained by this step, which was wet with ion-exchanged distilled water, was measured for a necessary amount (20 mL) with a graduated cylinder filled with ion-exchanged distilled water, and then the adsorbent was packed in a polypropylene column filled with ion-exchanged distilled water. Compressed air (1 kg/cm²) was fed into the column to force out a part of the filling liquid to give an adsorber containing an adsorbent in a wet state and having a ratio of a distance between the bottom of the packing part and a liquid surface of the filling liquid to a distance between the bottom of the packing part and the top of the adsorbent of 0.4, which was subjected to radiation sterilization.

### Comparative Example 1

The adsorbent obtained in Production Example 1 was immersed in ion-exchanged distilled water and degassed under reduced pressure. The adsorbent obtained by this step, which was wet with ion-exchanged distilled water, was measured for a necessary amount (20 mL) with a graduated cylinder filled with ion-exchanged distilled water, and then the adsorbent was packed in a polypropylene column filled with ion-exchanged distilled water to give an adsorber containing an adsorbent filled with a filling liquid, which was subjected to radiation sterilization.

### Comparative Example 2

The mixture of cation-exchange resin prepared in Production Example 1 (1) was dried at 110°C for 2 hr, and then the volume was measured (16 mL) with a graduated cylinder. The mixture was packed in a polypropylene column to give an adsorber containing an adsorbent in a dry state, which was subjected to radiation sterilization.

### Experimental Example 1

Bovine blood was flown through the adsorbers obtained in Examples and Comparative Example at a flow rate of 10 mL/min, and the inlet pressure of the column was measured with the lapse of time. The difference in the pressure between the start and upon liquid (400 mL) passage is shown in Table 1.

Each adsorbent (20 g) taken out from the adsorbers prepared in Examples and Comparative Example was immersed in 200 mL of distilled water, and after heating at 121°C for 20 min, filtrated through a glass filter, and measured for UV absorption spectrum of the filtrate. By determining the maximum absorbance, the amount of the eluate was evaluated. The results are shown in Table 1.

The value of maximum absorbance of UV absorption spectrum needs to be adjusted to less than 0.1 for safety as indicated in the Quality standard for Hemoperfusion adsorption columns (Standard of Japan Artificial Organ Industry Association): issued January 23, 1983).

**Table 1**

| | State of adsorbent | Ratio of filling liquid^{*1} | PHEMA coating | Inlet pressure difference on liquid flow (mmHg) | Maximum absorbance of filtrate |
|---|---|---|---|---|---|
| Example 1 | dry | 0 | Applied | 88 | 0.022 |
| Example 2 | wet | 0 | Applied | 21 | 0.051 |
| Example 3 | wet | 0.4 | Applied | 15 | 0.088 |
| Comparative Example 1 | wet | 1.0 | Applied | 9 | 0.113 |
| Comparative Example 2 | dry | 0 | None | 217 | 0.020 |

| | | | | | |
|---|---|---|---|---|---|
| *1 A ratio of a distance between the bottom of the packing part and a liquid surface of the filling liquid to a distance between the bottom of the packing part and the top of the adsorbent, wherein the adsorbent was packed in the packing part such that the top was horizontal. | | | | | |

From the results in Table 1 above, it is clear that, in Examples 1-3, in which an adsorbent coated with PHEMA was used, the difference in inlet pressure of each column was small as compared to Comparative Example 2, in which a dry adsorbent not coated with PHEMA was used, thus posing no risk of causing hemolysis. Moreover, it is clear from the results of maximum absorbance of the UV absorption spectrum of the filtrate that, in Examples 1-3, in which the adsorbent coated with PHEMA was not filled with a filling liquid, the maximum absorbance of any filtrate was less than 0.1, thus showing high safety with less eluate, but in Comparative Example 1, in which the adsorbent coated with PHEMA was filled with a filling liquid, eluate was intense.

### Examples 4-9

The Na salt type cation-exchange resin and Ca salt type cation-exchange resin prepared in the same manner as in Production Example 1 (1) were mixed at a ratio (volume ratio) shown in Table 2, and each ion-exchange resin (mixture, 20 mL) was measured with a graduated cylinder filled with ion-exchanged distilled water. Then the resin was packed in a polypropylene column filled with ion-exchanged distilled water. After packing, compressed air (1.0 kg/cm²) was fed for 1 min to give an adsorber containing an adsorbent in a wet state and having a packing part not filled with a filling liquid, which was subjected to radiation sterilization. Bovine blood was flown through this adsorber at a flow rate of 10 mL/min. The blood that passed through the adsorber was taken every minute for 10 min after the start and every 10 min thereafter, and plasma obtained by centrifugal separation and plasma obtained by centrifugal separation of the blood before passing through the adsorber were measured for sodium, potassium, calcium and chlorine ion concentrations, respectively. The sodium, potassium and chlorine ion concentrations were measured by an electrode method, and the calcium concentration was measured by an OCPC method, respectively (o-Cresolphthalein complexone method).

The sodium, potassium, calcium and chlorine ion concentrations before treatment by the adsorber were 157 mEq/L, 43 mEq/L, 7.3 mg/dL and 112 mEq/L, respectively. Changes in ion concentration of the blood taken 20 min later relative thereto are shown in Table 2.

**Table 2**

| | salt type (Na:Ca) | K | Ca | Na | Cl |
|---|---|---|---|---|---|
| Example 4 | 95:5 | -85.1% | +52.7% | +23.6% | 0% |
| Example 5 | 96:4 | -84.2% | +35.6% | +19.9% | 0% |
| Example 6 | 97:3 | -84.2% | +29.7% | +21.7% | 0% |
| Example 7 | 98:2 | -81.1% | +12.0% | +19.2% | -0.9% |
| Example 8 | 99:1 | -82.8% | +4.0% | +21.0% | 0% |
| Example 9 | 100:0 | -84.0% | -32.9% | +24.8% | 0% |

From the results of Table 2 above, it is clear that potassium ion was efficiently removed from the blood treated by the adsorber for body fluid treatment of the present invention.

### Industrial Applicability

According to the present invention, an adsorber for body fluid treatment can be provided, which permits only a small amount of eluate and which affords easy wetting and easy handling. In addition, an adsorber for body fluid treatment, which is capable of certainly removing potassium ion in the preserved blood for transfusion by adsorption, can be provided.

This application is based on a patent application No. 113643/2001 filed in Japan, the contents of which are hereby incorporated by reference.

## Claims

1. An adsorber for body fluid treatment packed with an adsorbent coated with a hydrophilic polymer, which comprises an adsorbent packing part not filled with a filling liquid.

2. The adsorber for body fluid treatment of claim 1, wherein the adsorbent is wet with a filling liquid but the adsorbent packing part is not filled with a filling liquid.

3. The adsorber for body fluid treatment of claim 2, wherein a ratio of a distance between the bottom of the packing part and a liquid surface of the filling liquid to a distance between the bottom of the packing part and the top of the adsorbent is not more than 0.8.

4. The adsorber for body fluid treatment of any of claims 1 to 3, wherein the adsorbent comprises two or more kinds of metal salt type cation-exchange resins that are different from each other.

5. The adsorber for body fluid treatment of claim 4, wherein the adsorbent comprises 90-99.9 parts by volume of a sodium salt type cation-exchange resin and 10-0.1 part by volume of a calcium salt type cation-exchange resin.

6. The adsorber for body fluid treatment of any of claims 1 to 5, which is an ion concentration controller of preserved blood for transfusion.
